# EUROPEAN PATENT APPLICATION

(11) **EP 4 847 131 A1**
(43) Date of publication of application: **02.12.2026**
(21) Application number: 25205568.6
(22) Date of filing: 30.09.2025
(51) Int. Cl.: B01J 23/89, B01J 37/02, B01J 37/18, C07C 39/04

(54) **TRIMETALLIC CATALYST AND PREPARING METHOD THEREOF AND METHOD FOR RECOVERING DIPHENOL COMPOUND**

(30) Priority: 26.05.2025 CN 202510684734
(71) Applicant: Swancor Regener Co., LTD, Nantou City, Nantou County 540028 (TW)
(72) Inventor: HUANG, Yun-Wen, 540028 Nantou City (TW); WANG, Meng-Wei, 540028 Nantou City (TW); KAO, Jun-Qi, 540028 Nantou City (TW); WU, Jing-Yun, 540028 Nantou City (TW); TSAI, Meng-Jung, 540028 Nantou City (TW); LEE, Pei-Rong, 540028 Nantou City (TW)
(74) Representative: Ter Meer und Partner mbB

(57) **Abstract**

Provided are a trimetallic catalyst and a preparation method thereof and a method for recovering a diphenol compound. The trimetallic catalyst includes a carrier and a first metal, a second metal and Ni that are loaded on the carrier. The first metal includes a transition metal other than Ni, Pd and Pt. The second metal includes Pd or Pt. The carrier includes CeO₂.

## Description

### BACKGROUND

### Technical Field

The invention relates to a trimetallic catalyst and a preparation method and a use thereof and an application, and particularly relates to a trimetallic catalyst for degrading an alkyl aryl ether-containing compound and a preparation method and a use thereof and a method for recovering a diphenol compound.

### Related Art

Epoxy resin is a very important thermosetting material in the current market, having characteristics such as low viscosity and good operability before curing, and is therefore commonly used in fiber-containing composite materials with high strength and lightweight needs, and is widely applied in fields such as adhesives, military industry, wind turbines, and printed circuit boards.

However, since a cured product generated after a thermosetting material has been cured has irreducibility, the cured product scraps and cured product waste generated during processing may only be incinerated or buried after physical crushing, making it difficult to recover and reuse. In addition, the foregoing waste treatment process may cause environmental pollution, so that the development of thermosetting materials faces difficulties. In the art methods for treating a waste epoxy resin cured product through chemical degradation have been developed. However, the prior art processes are related to various draw-backs. In particular, a chemical degradation process needs large amounts of solvent as well as strong acid or strong base, wherein in particular the use of strong acids or bases cause obstacles to purify a degradation product after the chemical degradation.

China Patent No. 111718515B discloses an epoxy resin cured product formed by bisphenol A (BPA) type epoxy resin and maleic anhydride, and a degradation system commonly configured by such epoxy resin cured product and water and trichloroacetic acid. A degradation reaction is performed on the degradation system at 170°C for 26 hours. Afterwards, through steps such as dissolution, filtration and distillation, a mixture including water, maleic acid, trichloroacetic acid and a degradation product of the epoxy resin cured product (a degradation rate of 95%) may be obtained. However, the degradation product of the epoxy resin cured product may not be definitely extracted from the mixture. Therefore, it is difficult to apply a high-value application on the degradation product.

China Patent No. 114479177B discloses a method for degrading an epoxy resin cured product formed by bisphenol A epoxy resin and acid anhydride using dimethylformamide and ethylene glycol. After degradation, a solvent is recovered in a distillation manner. An oligomer generated by degradation may serve as a raw material to prepare new epoxy resin again. The foregoing oligomer is a polyol compound.

Kyoko et al. published a paper in 2024 regarding a bimetallic catalyst configured to degrade an epoxy resin cured product titled "Hydrogenolysis of epoxy resins by a reusable CeO2-supported Ni-Pd bimetallic catalyst toward recycling of epoxy thermosets". The activated NiPd/CeO₂ bimetallic catalyst may degrade an alkyl aryl ether structure in the epoxy resin cured product, ultimately obtaining bisphenol A (BPA), 4-isopropylphenol, and phenol. However, although the method may recover fibers from a fiber-containing composite material, an alkyl bonding between two phenols in a bisphenol A structure is also destroyed while degrading the alkyl aryl ether structure, so that two by-products, 4-isopropylphenol and phenol, are obtained. That is to say, in the method, the catalyst used has a poor selectivity for bond-breaking position, thereby difficult to obtain a high-purity diphenol compound.

Diphenol compounds, such as bisphenol A, are indispensable chemical raw materials in today's industry. Tens of thousands of tons of bisphenol A are used annually for the production of bisphenol A type epoxy resins, such as bisphenol A diglycidyl ether (DGEBA). Therefore, if the diphenol compounds in epoxy resins may be recovered and reused in an epoxy resin manufacturing process, it can significantly reduce the amount of epoxy resin waste treatment and contribute to carbon reduction needs. Consequently, it was an object of the present invention to overcome the drawbacks of the prior art and to provide a catalyst and a method for the recovery of diphenol compounds, in particular to provide catalyst and a method for the recovery of diphenol compounds from alkyl aryl ether-containing compounds. The objects have been unexpectedly solved by the trimetallic catalyst and the methods of the present invention.

### SUMMARY

The invention provides a trimetallic catalyst, a preparation method and a use thereof and a method for recovering a diphenol compound. The trimetallic catalyst has a high selectivity for bond-breaking position, so that a high-purity diphenol compound may be extracted from a degradation product, and the trimetallic catalyst may be recovered and reused after degradation, which meets the spirit of economic efficiency and sustainable usage.

The trimetallic catalyst of the invention includes a carrier and a first metal M1, a second metal M2 and Ni that are loaded on the carrier. The M1 includes a transition metal other than Ni, Pd and Pt. The M2 includes Pd or Pt. The carrier includes CeO₂.

In an embodiment of the trimetallic catalyst of the invention, the M1 is Fe, Mn, Co, or Cu.

In a preferred embodiment of the trimetallic catalyst of the invention, the M1 is Fe, and the M2 is Pd or Pt. In a more preferred embodiment of the trimetallic catalyst of the invention, the M1 is Fe and the M2 is Pd. In an alternative more preferred embodiment of the trimetallic catalyst of the invention, the M1 is Fe and the M2 is Pt.

In an embodiment of the trimetallic catalyst of the invention, the M1 is Mn, and the M2 is Pd.

In an embodiment of the trimetallic catalyst of the invention, the M1 is Co or Cu, and the M2 is Pd. In a more preferred embodiment of the trimetallic catalyst of the invention, the M1 is Co and the M2 is Pd. In an alternative more preferred embodiment of the catalyst of the invention, the M1 is Cu and the M2 is Pd.

In an embodiment of the trimetallic catalyst of the invention, the trimetallic catalyst is represented by Formula (1), and 0<x≦2, 0<y≦2, and 0<z≦2,

(M1)ₓ(Ni)_{y}(M2)_{z}/CeO₂ Formula (1).

A method of preparing the foregoing trimetallic catalyst of the invention includes the following steps. A compound containing the M1, a compound containing the M2, and a Ni-containing compound are dissolved in water to obtain a mixed solution. CeO₂ serving as the carrier is added to the mixed solution to perform reaction.

In an embodiment of the method of preparing the trimetallic catalyst of the invention, the trimetallic catalyst is represented by Formula (1), and x, y, and z respectively represent a millimole number of each of the M1, the M2, and Ni in the compound containing the M1, the compound containing the M2, and the Ni-containing compound used per 23.3 millimoles of a usage amount of the carrier, and 0<x≦2, 0<y≦2, and 0<z≦2,

(M1)ₓ(Ni)_{y}(M2)_{z}/CeO₂ Formula (1).

A method for recovering the diphenol compound of the invention includes the following steps. An activation treatment is performed on the foregoing trimetallic catalyst to obtain an activated trimetallic catalyst. The activated trimetallic catalyst is used to perform a degradation treatment on an alkyl aryl ether-containing compound to obtain a mixture solution. The mixture solution is filtered to separate a filtrate and the trimetallic catalyst. Distillation is performed on the filtrate to obtain the diphenol compound.

In an embodiment of the method for recovering the diphenol compound of the invention, the activation treatment includes the following steps. The trimetallic catalyst is added to a high-pressure reactor, vacuum extraction is performed, and then hydrogen is introduced. A first heat treatment is performed to increase a temperature to an activation temperature of the trimetallic catalyst, wherein a temperature of the first heat treatment is preferably between 130°C and 300°C.

In an embodiment of the method for recovering the diphenol compound of the invention, the degradation treatment includes the following steps. The alkyl aryl ether-containing compound, the activated trimetallic catalyst, and a solvent are added to a high-pressure reactor, vacuum extraction is performed, and then hydrogen is introduced. A second heat treatment is performed to increase a temperature to a degradation temperature of the alkyl aryl ether-containing compound, wherein the second heat treatment is between 150°C and 240°C, preferably between 170°C and 220 °C.

In an embodiment of the method for recovering the diphenol compound of the invention, the alkyl aryl ether-containing compound includes a monomer containing an alkyl aryl ether structure, an oligomer containing an alkyl aryl ether structure, a polymer containing an alkyl aryl ether structure, or a combination thereof.

In an embodiment of the method for recovering the diphenol compound of the invention, the polymer containing an alkyl aryl ether structure includes a thermosetting polymer or a thermoplastic polymer.

In an embodiment of the method for recovering the diphenol compound of the invention, in the degradation treatment, taking a total amount of the alkyl aryl ether-containing compound as a reference, the activated trimetallic catalyst is added in an amount between 20 wt% and 70 wt%, preferably between 25 wt.% and 50 wt.%.

A further aspect of the invention is the use of the foregoing trimetallic catalyst for the recovery of a diphenol compound from an alkyl aryl ether-containing compound.

Based on the above, the trimetallic catalyst of the invention may be configured to degrade a compound containing an alkyl aryl ether structure. The high-purity diphenol compound may be recovered from a degraded mixture. In addition, the trimetallic catalyst of the invention may be recovered and reactivated for usage after use. The recovered diphenol compound may be made into an epoxy resin or a related chemical through a chemical reaction, so that the purpose of waste recycling and reuse can be achieved.

### BRIEF DESCRIPTION OF THE DRAWINGS

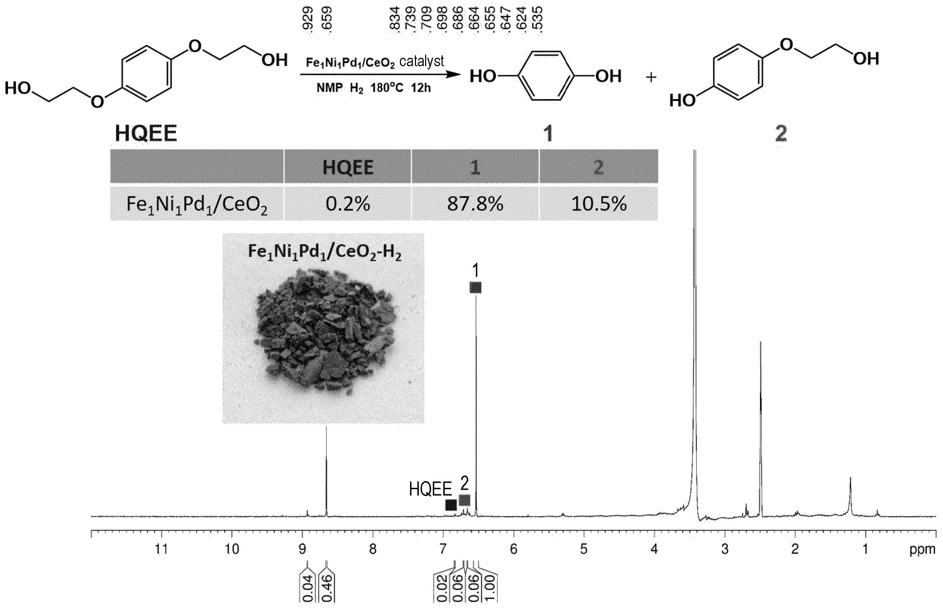

FIGURE 1 is a ¹H nuclear magnetic resonance (¹H NMR) spectrum of the experimental results of Embodiment 1.

### DESCRIPTION OF THE EMBODIMENTS

Herein, a range indicated by "one value to another value" is a general representation which avoids enumerating all values in the range in the specification. Therefore, the record of a specific value range, any number within this numerical range and any smaller numerical range bounded by any number within that numerical range is contemplated as if such any number and such smaller numerical ranges were expressly written in the specification.

In addition, herein, "oligomer" generally refers to a polymer with a weight average molecular weight (Mw) lower than 5000. "Polymer" generally refers to a polymer with a weight average molecular weight of 5000 or above. In addition, "polymer" may be a thermosetting polymer or a thermoplastic polymer. The thermosetting polymer, such as an epoxy resin cured product (a gel block) herein, refers to a polymer having a crosslinked structure, which is usually insoluble in an organic solvent and therefore difficult to define a molecular weight. The foregoing weight average molecular weight is a value measured using a gel permeation chromatography (GPC) method and is defined as a polystyrene (PS) conversion value. Herein, regarding the weight average molecular weight, it is determined by using, for example, HLC-8220GPC (manufactured by TOSOH CORPORATION) and connecting protection columns HZ-L, TSKgel Super HZM-M, TSKgel Super HZ4000, TSKgel Super HZ3000, and TSKgel Super HZ2000 (all manufactured by TOSOH CORPORATION) in series to serve as a column. Unless otherwise described, the foregoing weight average molecular weight is measured using tetrahydrofuran (THF) as an eluent. A detector with an ultraviolet wavelength of 254 nm is used in the GPC measurement.

In addition, in the process of preparing the trimetallic catalyst of the invention, since metals in various metal-containing reactants and a carrier are not consumed during a reaction process, an amount of metals in the various metal-containing reactants used and an amount of carrier are substantially equal to a content of each metal and a content of carrier in a catalyst product. That is to say, the amount of metals in the various metal-containing reactants used and the amount of carrier may be regarded as the content of each metal and the content of carrier in the catalyst product.

The invention provides a trimetallic catalyst, which is adapted to perform a degradation treatment on an alkyl aryl ether-containing compound. The trimetallic catalyst has a high bond-breaking position selectivity, therefore a high-purity diphenol compound may be extracted after the degradation treatment, effectively improving a recovery rate of the diphenol compound. In addition, after the degradation treatment, the trimetallic catalyst of the invention may be recovered and reused to meet the spirit of economic efficiency and sustainable usage. The following will provide detailed descriptions of the trimetallic catalyst of the invention and the degradation treatment of the alkyl aryl ether-containing compound using the same.

### [Trimetallic Catalyst]

The trimetallic catalyst of the invention includes a carrier and a first metal M1, a second metal M2, and Ni that are loaded on the carrier, where the M1 includes a transition metal other than Ni, Pd and Pt, the M2 includes Pd or Pt, and the carrier includes CeO₂. Preferably, the M1 may be Fe, Mn, Co, or Cu.

The trimetallic catalyst of the invention can be represented by Formula (1), where 0<x≦2, 0<y≦2, and 0<z≦2. That is, x, y, and z may each independently be 2 or any number less than 2 and greater than 0,

(M1)ₓ(Ni)_{y}(M2)_{z}/CeO₂ Formula (1).

In addition, in Formula (1), each metal included in the catalyst is described on the left side of "/". The carrier loading the foregoing metals is described on the right side of "/". "/" is configured to separate each metal and the carrier. A corresponding content ratio relationship is separately described in the following paragraphs of the specification.

In the trimetallic catalyst of the invention, there is preferably a specific ratio range between an amount of the carrier and an amount of each of the M1, the M2, and Ni. For example, when the trimetallic catalyst of the invention is prepared, when a usage amount of the carrier is 23.3 mmol, in the reactants used, the amount of each of the M1, the M2, and Ni is greater than zero and not exceeding 2 mmol. Therefore, in Formula (1), x, y, and z may respectively represent a content (millimoles) of each of the M1, the M2, and Ni in a compound containing the M1, a compound containing the M2, and a Ni-containing compound used per 23.3 millimoles of a usage amount of the carrier. The definition of the word "per" is that when the usage amount (millimoles) of the carrier is scaled up or down, the content (millimoles) of each of the M1, the M2, and Ni is also proportionally scaled up or down. Therefore, the invention is not limited to a fixed use of 23.3 millimoles of the carrier.

In some exemplary embodiments, the trimetallic catalyst of the invention may be Fe₁Ni₁Pd₁/CeO₂, Fe₁Ni₁Pt₁/CeO₂, Co₁Ni₁Pd₁/CeO₂, Cu₁Ni₁Pd₁/CeO₂, Mn₁Ni₁Pd₁/CeO₂, Fe_{0.67}Ni_{0.67}Pd_{0.67}/CeO₂, Fe₁Ni₁Pd_{0.5}/CeO₂, Fe₂Ni₂Pd₁/CeO₂, Fe₁Ni₂Pd₁/CeO₂, Fe₂Ni₁Pd₁/CeO₂, Fe₁Ni₁Pd₂/CeO₂, Fe₁Ni_{0.5}Pd₁/CeO₂, etc. However, the invention is not limited thereto.

In a preferred embodiment, the trimetallic catalyst of the invention may be (Fe)ₓ(Ni)_{y}(Pd)_{z}/CeO₂, where 0<x≦2, 0<y≦2, and 0<z≦2. Examples are Fe₁Ni₁Pd₁/CeO₂, Fe_{0.67}Ni_{0.67}Pd_{0.67}/CeO₂, Fe₁Ni₁Pd_{0.5}/CeO₂, Fe₂Ni₂Pd₁/CeO₂, Fe₁Ni₂Pd₁/CeO₂, Fe₂Ni₁Pd₁/CeO₂, Fe₁Ni₁Pd₂/CeO₂, Fe₁Ni_{0.5}Pd₁/CeO₂.

In a more preferred embodiment, the trimetallic catalyst of the invention may be Fe₁Ni₁Pd₁/CeO₂, Fe_{0.67}Ni_{0.67}Pd_{0.67}/CeO₂, Fe₁Ni₁Pd_{0.5}/CeO₂, Fe₂Ni₂Pd₁/CeO₂, Fe₁Ni₂Pd₁/CeO₂, Fe₂Ni₁Pd₁/CeO₂, more preferably Fe₁Ni₁Pd₁/CeO₂, Fe₂Ni₂Pd₁/CeO₂, and Fe₁Ni₂Pd₁/CeO₂, in particular Fe₁Ni₁Pd₁/CeO₂.

### [Preparation of the Trimetallic Catalyst]

The trimetallic catalyst of the invention may be prepared through the following steps.

First, the compound containing the first metal M1, the compound containing the second metal M2, and the Ni-containing compound can be dissolved in water (such as pure water) to obtain a mixed solution containing metal ions of the M1, the M2, and Ni. Next, CeO₂ serving as the carrier can be added to the mixed solution. Then, the mixed solution can be stirred, for example at room temperature (25 °C), e.g. for 24 hours to perform reaction. Afterwards, the mixed solution can be filtered to obtain a powder product. The powder product is the trimetallic catalyst of the invention. The following synthesis examples are provided to further describe the method of preparing the trimetallic catalyst of the invention.

### Synthesis Example 1:

81 mg (0.50 mmol) of iron trichloride, 75 mg (1.00 mmol) of potassium chloride, 64.8 mg (0.50 mmol) of nickel chloride, and 88.7 mg (0.50 mmol) of palladium chloride were dissolved in 60 mL of pure water, and stirred at room temperature to dissolve to form a mixed solution. Then, 2.005 g (11.65 mmol) of cerium oxide was added to the mixed solution, and stirred at room temperature for 24 hours. Next, the mixed solution was filtered, and the remaining solid after filtration is rinsed with pure water. The rinsed solid was dried at room temperature to obtain a product Fe₁Ni₁Pd₁/CeO₂ (1.854 g, a yield about 87.6%).

### Synthesis Example 2:

The preparation steps were the same as Synthesis Example 1, except that 88.7 mg (0.50 mmol) of palladium chloride was replaced with 133.0 mg (0.50 mmol) of platinum dichloride to obtain a product Fe₁Ni₁Pt₁/CeO₂ (2.103 g, a yield about 97.4%).

### Synthesis Example 3:

The preparation steps were the same as Synthesis Example 1, except that 81.0 mg (0.50 mmol) of iron chloride was replaced with 64.92 mg (0.50 mmol) of cobalt chloride to obtain a product Co₁Ni₁Pd₁/CeO₂ (2.008 g, a yield about 94.9%).

### Synthesis Example 4:

The preparation steps were the same as Synthesis Example 1, except that 81.0 mg (0.50 mmol) of iron chloride was replaced with 67.2 mg (0.50 mmol) of copper chloride to obtain a product Cu₁Ni₁Pd₁/CeO₂ (2.033 g, a yield about 96.0%).

### Synthesis Example 5:

The preparation steps were the same as Synthesis Example 1, except that 81.0 mg (0.50 mmol) of iron chloride was replaced with 62.92 mg (0.50 mmol) of manganese chloride to obtain a product Mn₁Ni₁Pd₁/CeO₂ (2.027 g, a yield about 95.9%).

### Synthesis Example 6:

The preparation steps were the same as Synthesis Example 1, except that a usage amount of nickel chloride was increased to 129.60 mg (1.00 mmol) to obtain a product Fe₁Ni₂Pd₁/CeO₂ (2.099 g, a yield about 97.9%).

### Synthesis Example 7:

The preparation steps were the same as Synthesis Example 1, except that a usage amount of iron trichloride was increased to 162.2 mg (1.00 mmol) to obtain a product Fe₂Ni₁Pd₁/CeO₂ (2.101 g, a yield about 98.0%).

### Synthesis Example 8:

The preparation steps were the same as Synthesis Example 1, except that a usage amount of palladium chloride was increased to 177.3 mg (1.00 mmol) to obtain a product Fe₁Ni₁Pd₂/CeO₂ (2.081 g, a yield about 96.0%).

### Synthesis Example 9:

The preparation steps were the same as Synthesis Example 1, except that the usage amount of iron trichloride was decreased to 54.2 mg (0.334 mmol), the usage amount of nickel chloride was decreased to 43.4 mg (0.334 mmol), and the usage amount of palladium chloride was decreased to 59.36 mg (0.334 mmol) to obtain a product Fe_{0.67}Ni_{0.67}Pd_{0.67}/CeO₂ (1.900 g, a yield about 91.8%).

### Synthesis Example 10:

The preparation steps were the same as Synthesis Example 1, except that the usage amount of palladium chloride was decreased to 44.33 mg (0.25 mmol) to obtain a product Fe₁Ni₁Pd_{0.5}/CeO₂ (2.081 g, a yield about 92.7%).

### Synthesis Example 11:

The preparation steps were the same as Synthesis Example 1, except that the usage amount of iron trichloride was increased to 163.0 mg (1.00 mmol), and the usage amount of nickel chloride was increased to 129.6 mg (1.00 mmol) to obtain a product Fe₂Ni₂Pd₁/CeO₂ (2.012 g, a yield about 92.0%).

### Synthesis Example 12:

The preparation steps were the same as Synthesis Example 1, except that the usage amount of nickel chloride was decreased to 33.0 mg (0.25 mmol) to obtain a product Fe₁Ni_{0.5}Pd₁/CeO₂ (1.918 g, a yield about 90.2%).

In addition, in the following comparative synthesis examples, the same or similar preparation steps were utilized to prepare a metallic catalyst.

### Comparative Synthesis Example 1

74.9 mg (1.00 mmol) of potassium chloride, 65.8 mg (0.50 mmol) of nickel chloride and 88.7 mg (0.50 mmol) of palladium chloride were dissolved in 60 mL of pure water, and stirred at room temperature to dissolve to form a mixed solution. Then, 2.005 g (11.65 mmol) of cerium oxide was added to the mixed solution, and stirred at room temperature for 24 hours. After the reaction was complete, the mixed solution was filtered, and the remaining solid after filtration was rinsed with pure water. The rinsed solid was dried at room temperature to obtain a product Ni₁Pd₁/CeO₂ (2.046 g, a yield 98.0%).

### Comparative Synthesis Example 2:

The preparation steps were the same as Synthesis Example 1, except that 2.005 g of cerium oxide was replaced with 1.998 g of zinc oxide to obtain a product Fe₁Ni₁Pd₁/ZnO (2.024 g, a yield about 95.9%).

### Comparative Synthesis Example 3:

The preparation steps were the same as Synthesis Example 1, except that 2.005 g of cerium oxide was replaced with 2.002 g of nickel oxide to obtain a product Fe₁Ni₁Pd₁/NiO (2.013 g, a yield about 95.3%).

### Comparative Synthesis Example 4:

The preparation steps were the same as Synthesis Example 1, except that 2.005 g of cerium oxide was replaced with 2.006 g of magnesium oxide to obtain a product Fe₁Ni₁Pd₁/MgO (2.003 g, a yield about 94.6%).

### Comparative Synthesis Example 5:

The preparation steps were the same as Synthesis Example 1, except that 2.005 g of cerium oxide was replaced with 2.003 g of titanium dioxide to obtain a product Fe₁Ni₁Pd₁/TiO₂ (2.001 g, a yield about 94.7%).

### Comparative Synthesis Example 6:

The preparation steps were the same as Synthesis Example 1, except that 2.005 g of cerium oxide was replaced with 1.997 g of aluminum oxide to obtain a product Fe₁Ni₁Pd₁/Al₂O₃ (2.002 g, a yield about 95.0%).

### Comparative Synthesis Example 7:

The preparation steps were the same as Synthesis Example 1, except that 2.005 g of cerium oxide was replaced with 1.999 g of zirconium dioxide to obtain a product Fe₁Ni₁Pd₁/ZrO₂ (1.997 g, a yield about 94.6%).

### Comparative Synthesis Example 8:

The preparation steps were the same as Comparative Synthesis Example 1, except that 65.8 mg (0.50 mmol) of nickel chloride was replaced with 81.1 mg (0.50 mmol) of iron chloride to obtain a product Fe₁Pd₁/CeO₂ (2.003 g, a yield about 96.0%).

### Comparative Synthesis Example 9:

The preparation steps were the same as Comparative Synthesis Example 1, except that 65.8 mg (0.50 mmol) of nickel chloride was replaced with 67.2 mg (0.50 mmol) of copper chloride to obtain a product Cu₁Pd₁/CeO₂ (1.985 g, a yield about 95.0%).

### Comparative Synthesis Example 10:

The preparation steps were the same as Comparative Synthesis Example 1, except that 65.8 mg (0.50 mmol) of nickel chloride was replaced with 64.9 mg (0.50 mmol) of cobalt chloride to obtain a product Co₁Pd₁/CeO₂ (2.001 g, a yield about 95.8%).

### Comparative Synthesis Example 11:

The preparation steps were the same as Comparative Synthesis Example 1, except that 65.8 mg (0.50 mmol) of nickel chloride was replaced with 62.92 mg (0.50 mmol) of manganese chloride to obtain a product Mn₁Pd₁/CeO₂ (1.995 g, a yield about 95.8%).

### Comparative Synthesis Example 12:

The preparation steps were the same as Comparative Synthesis Example 1, except that 88.7 mg (0.50 mmol) of palladium chloride was replaced with 133.0 mg (0.50 mmol) of platinum dichloride to obtain a product Ni₁Pt₁/CeO₂ (2.088 g, a yield about 97.9%).

### Comparative Synthesis Example 13:

The preparation steps were the same as Synthesis Example 1, except that the usage amount of nickel chloride was increased to 324 mg (2.50 mmol) to obtain a product Fe₁Ni₅Pd₁/CeO₂ (2.093 g, a yield about 93.7%).

### Comparative Synthesis Example 14:

The preparation steps were the same as Synthesis Example 1, except that the usage amount of iron trichloride was increased to 405.5 mg (2.50 mmol) to obtain a product Fe₅Ni₁Pd₁/CeO₂ (2.103 g, a yield about 94.4%).

### Comparative Synthesis Example 15:

The preparation steps were the same as Synthesis Example 1, except that the usage amount of palladium chloride was increased to 443.325 mg (2.50 mmol) to obtain a product Fe₁Ni₁Pd₅/CeO₂ (2.111 g, a yield about 90.7%).

### [Recovery of the Diphenol Compound]

The trimetallic catalyst of the invention may be applied to degrade an alkyl aryl ether-containing compound and further recover the high-purity diphenol compound. The alkyl aryl ether-containing compound may be a monomer containing an alkyl aryl ether structure, an oligomer containing an alkyl aryl ether structure, a polymer containing an alkyl aryl ether structure, or a combination thereof. In addition, the trimetallic catalyst of the invention may be recovered and reactivated for usage after use.

First, an activation treatment is performed on the trimetallic catalyst of the invention to obtain an activated trimetallic catalyst. In an embodiment, the foregoing trimetallic catalyst may be added to a high-pressure reactor, vacuum extraction is performed, and hydrogen is introduced. The high-pressure reactor is, for example, a novoclave stainless steel high-pressure reaction equipment from Buchiglasuster. Then, a heat treatment can be performed to increase a temperature to an activation temperature of the trimetallic catalyst. A temperature of the foregoing heat treatment is, for example, between 130°C and 300°C, preferably between 150°C and 240°C. When the temperature of the heat treatment is lower than 130°C, a problem of uneven catalyst activation may be caused. When the temperature of the heat treatment is higher than 300°C, industrial production efficiency is not met, and safety risks increase significantly.

Next, the activated trimetallic catalyst can be used to perform a degradation reaction on the alkyl aryl ether-containing compound. After the degradation reaction is complete and the temperature is reduced to room temperature, a mixture solution is obtained. In the mixture solution, taking a total amount of the alkyl aryl ether-containing compound as a reference, the activated trimetallic catalyst can added in an amount, for example, between 20 wt% and 70 wt%, preferably between 25 wt% and 50 wt%. When the amount of the activated trimetallic catalyst added in is less than 20 wt%, the efficiency of the degradation treatment may be too low. When the amount of the activated trimetallic catalyst added in is more than 70 wt%, the efficiency of the degradation treatment may not be significantly improved, and causes a substantial increase in cost.

In some embodiments, the alkyl aryl ether-containing compound, a solvent, the trimetallic catalyst, and a magnet may be added to the foregoing high-pressure reactor, vacuum extraction is performed, and then hydrogen is introduced, and heating is further performed to increase a temperature to a degradation temperature of the alkyl aryl ether-containing compound to perform the degradation reaction. After the degradation reaction is complete and the temperature is reduced to room temperature, the foregoing mixture solution may be obtained. The foregoing solvent may be N-methylpyrrolidone, N,N-dimethylacetamide, dimethylformamide, 1,3-dimethyl-2-imidazolidinone, 1,4-dioxane or amine solvents. A temperature of the foregoing heat treatment is, for example, between 150°C and 240°C, preferably between 180°C and 200°C. When the temperature of the heat treatment is lower than 150°C, the degradation reaction can be incomplete. When the temperature of the heat treatment exceeds 300°C, fewer solvents can be chosen, making the process more challenging.

Then, the mixture solution is filtered to obtain a filtrate containing a degradation product. The solid trimetallic catalyst may be recovered at the same time.

Afterwards, distillation is performed on the filtrate is distilled to obtain the diphenol compound.

The method for recovering the diphenol compound of the invention will be described below with embodiments and comparative examples.

### [Alkyl Aryl Ether-Containing Compound]

Equivalent amounts of a BPA-type epoxy resin (BE188, purchased from Chang Chun Plastics Co., Ltd., epoxy equivalent of 188 g/eq) and hexahydrophthalic anhydride (HHPA) serving as a curing agent were mixed at a temperature of 60°C. Next, based on an amount of HHPA, 1 wt% of 2-ethyl-4-methylimidazole (2E4MI) was added and defoamed at a temperature of 60°C to prepare a mixture. Then, the mixture was poured into an aluminum pan and placed in a hot air circulation oven to perform a curing reaction with a stepwise temperature increase procedure (130°C/2 hours, 150°C/3 hours, and 190°C/2 hours) to form an epoxy resin cured product (a gel block). Next, the epoxy resin cured product (the gel block) was taken out and soaked in monoethanolamine (MEA) with a weight ratio of 1:10, and the temperature is increased to 135°C to perform a degradation reaction. After the epoxy cured product (the gel block) was completely dissolved, MEA was distilled under reduced pressure to obtain a viscous reddish-brown crude product. Then, acetone was used for washing, and gas extraction filtration was performed to obtain a filtrate. Afterwards, the filtrate was distilled under reduced pressure to remove acetone to obtain a refined oligomer containing an alkyl aryl ether structure (as shown in Formula (A) below), which serves as a target structure for degrading and extracting the diphenol compound, where n is a positive number greater than 0 and less than or equal to 70, preferably greater than 5 and less than 65, more preferably greater than 10 and less than 60, in particular greater than 15 and less than 55.

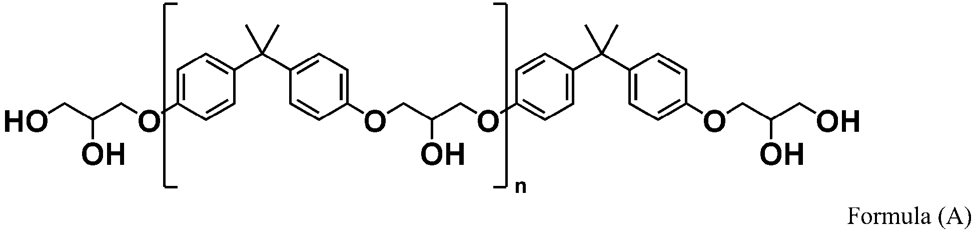

### [Epoxy Cured Product]

### Gel Block 1

Equivalent amounts of the BPA-type epoxy resin (BE188, epoxy equivalent of 188 g/eq) and HHPA were mixed at a temperature of 60°C. Next, based on an amount of HHPA, 1 wt% of 2E4MI was added and defoamed at a temperature of 60°C to prepare a mixture. Then, the mixture was poured into an aluminum pan and placed in a hot air circulation oven to perform a curing reaction with a stepwise temperature increase procedure (130°C/2 hours, 150°C/3 hours, and 190°C/2 hours) to form an epoxy resin cured product (a gel block), obtaining a gel block 1.

### Gel Block 2

Equivalent amounts of the BPA-type epoxy resin (BE188, epoxy equivalent of 188 g/eq) and isophorone diamine (IPDA) were mixed at room temperature and defoamed to prepare a mixture. Then, the mixture was poured into an aluminum pan and placed in a hot air circulation oven to perform a curing reaction with 80°C/24 hours to form an epoxy resin cured product (a gel block), obtaining a gel block 2.

### [Embodiment 1 and Comparative Example 1 to Comparative Example 8]

Hydroquinone bis(2-hydroxyethyl)ether (HQEE) was taken to serve as an alkyl aryl ether-containing compound of a monomer containing alkyl aryl ether functional groups, which generates hydroquinone and a monophenol monomer after the alkyl aryl ether functional group is cut. Reaction Formula (I) is as follows.

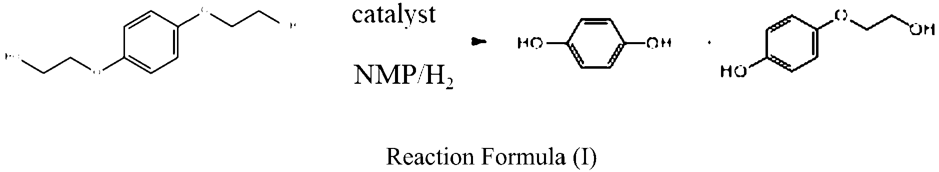

According to the experimental results, a conversion rate was calculated with a residual amount of HQEE in a ¹H nuclear magnetic resonance (¹H NMR) spectrum. A recovery rate of the diphenol compound is calculated according to a yield of the diphenol compound in the degradation product to perform a comparison of the degradation effects of the foregoing different catalysts. The following embodiments and comparative examples are provided to further describe the degradation reaction of the invention.

Embodiment 1 and Comparative Example 1 to Comparative Example 8 have the same implementation steps with different catalysts used, which are comprehensively described as follows.

Before the degradation reaction was performed, an activation treatment was performed on a synthesized catalyst. The synthesized catalyst was added to a high-pressure reactor, vacuum extraction was performed, and then 1 atmosphere (atm) of hydrogen was introduced. Then, heating was performed at a temperature of 150°C for 30 minutes to perform an activation. After the activation was complete, the temperature was allowed to return to room temperature to obtain an activated catalyst.

Next, the degradation reaction was performed. 200 milligrams of HQEE, 2 milliliters of N-methylpyrrolidinone (NMP), and a magnet were added to the foregoing high-pressure reactor containing the catalyst, deoxygenation and dehydration were performed, vacuum extraction was performed, and then 1 atm of hydrogen was introduced. Then, heating was performed (except Comparative Example 8 was heated to 148°C, all others are heated to 180°C) and stirred for 12 hours. After the reaction was complete, the temperature was allowed to drop to room temperature to obtain a mixture solution. Next, the mixture solution was filtered, and the catalyst was recovered. Afterwards, a filtrate generated after the mixture solution was filtered was distilled and purified to obtain a degradation product. The catalyst types, reaction conditions, and results used in Embodiment 1 and Comparative Example 1 to Comparative Example 8 are shown in Table 1 below.

**Table 1**

| | Catalyst | | Usage Amount (mg) | Degradation Temperature (°C) | Conversion Rate (%) | Diphenol Compound Recovery Rate (%) |
|---|---|---|---|---|---|---|
| Embodiment 1 | Synthesis Example 1 | Fe₁Ni₁Pd₁/CeO₂ | 100 | 180 | 98 | 88 |
| Comparative Example 1 | Comparative Synthesis Example 1 | Ni₁Pd₁/CeO₂ | 101.2 | | 90 | 85 |
| Comparative Example 2 | Comparative Synthesis Example 2 | Fe₁Ni₁Pd₁/ZnO | 100.5 | | 1 | 0 |
| Comparative Example 3 | Comparative Synthesis Example 3 | Fe₁Ni₁Pd₁/NiO | 99.8 | | 2 | 0 |
| Comparative Example 4 | Comparative Synthesis Example 4 | Fe₁Ni₁Pd₁/MgO | 101.1 | | 42 | 0 |
| Comparative Example 5 | Comparative Synthesis Example 5 | Fe₁Ni₁Pd₁/TiO₂ | 100.1 | | 20 | 0 |
| Comparative Example 6 | Comparative Synthesis Example 6 | Fe₁Ni₁Pd₁/Al₂O₃ | 100 | | 12 | 0 |
| Comparative Example 7 | Comparative Synthesis Example 7 | Fe₁Ni₁Pd₁/ZrO₂ | 100.3 | | 4 | 0 |
| Comparative Example 8 | Comparative Synthesis Example 1 | Ni₁Pd₁/CeO₂ | 101.2 | 148 | 0 | 0 |

The manner for calculating the conversion rate from the residual amount of HQEE in the ¹H NMR spectrum is as follows. Taking Embodiment 1 as an example, as shown in FIGURE 1, HQEE may generate a monophenol structure 1 and a bisphenol structure 2 after cracking. The benzene ring characteristic peaks of HQEE, the monophenol structure 1, and the bisphenol structure 2 in the ¹H NMR spectrum do not overlap. Structurally, since a hydrogen ratio on the benzene rings of the three is 1:1:1, the conversion rate may be proportionally calculated by integral values of the benzene ring characteristic peak areas of the three in the ¹H NMR spectrum.

The conversion rate calculation formula is: (an integral value of the benzene ring characteristic peak area of the monophenol structure 1 + an integral value of the benzene ring characteristic peak area of the bisphenol structure 2)/a sum of integral values of the benzene ring characteristic peak areas of the three × 100%

The diphenol compound recovery rate calculation formula is: (an integral value of the benzene ring characteristic peak area of the bisphenol structure 2)/a sum of integral values of the benzene ring characteristic peak areas of HQEE, the monophenol structure 1, and the bisphenol structure 2 × 100%

For example, in Embodiment 1, according to the ¹H NMR spectrum, the sum of the integral values of the benzene ring characteristic peak areas of HQEE, the monophenol structure 1, and the bisphenol structure 2 is 1+0.06+0.06+0.02=1.14. The integral value of the benzene ring characteristic peak area of the monophenol structure 1 is 0.06+0.06=0.12. The integral value of the benzene ring characteristic peak area of the bisphenol structure 2 is 1. Therefore, the conversion rate may be calculated as (0.12+1)/1.14×100%=98%. The bisphenol recovery rate is 1/1.14×100%=88%.

It can be seen from Table 1 that for HQEE serving as an alkyl aryl ether-containing compound, in the same degradation condition, compared with Comparative Example 1 utilizing a bimetallic catalyst, Embodiment 1 utilizing the trimetallic catalyst of the invention has a higher conversion rate and has a higher diphenol compound recovery rate.

In addition, when three identical metal elements are used to manufacture the trimetallic catalyst, replacing the carrier CeO₂ with different metal oxides does not show similar effects (Comparative Example 2 to Comparative Example 7). Although the conversion rate may barely reach 42% when MgO is used as the carrier, the product obtained was almost a monophenol compound, so that the diphenol compound recovery rate is nearly 0%. Therefore, Comparative Example 2 to Comparative Example 7 may not recover the diphenol compound.

In addition, it can be seen from the results of Embodiment 1 and Comparative Example 8, even the used catalyst is the same, when the degradation reaction temperature is lowered to be less than 150°C (148°C), the reaction may not be driven to occur. The conversion rate and the recovery rate are close to 0.

### [Embodiment 2 to Embodiment 15 and Comparative Example 9 to Comparative Example 18]

The oligomer containing an alkyl aryl ether structure of the foregoing Formula (A) is taken as the alkyl aryl ether-containing compound, which generates a bisphenol A after the alkyl aryl ether functional structure is cut. The bisphenol A might further break due to degradation, resulting in the generation of by-products such as phenol and 4-isopropylphenol. Reaction Formula (II) is as follows, where n is a positive number greater than 0 and less than or equal to 70, preferably greater than 5 and less than 65, more preferably greater than 10 and less than 60, in particular greater than 15 and less than 55.

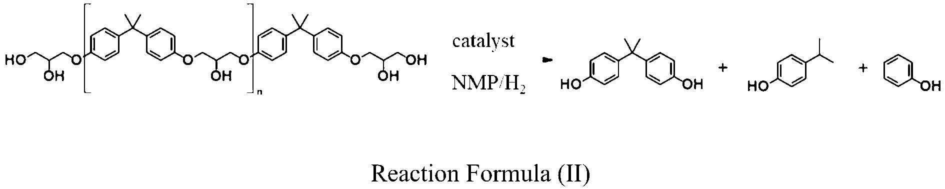

According to the experimental results, a conversion rate may be calculated with a residual amount of the oligomer containing an alkyl aryl ether structure of Formula (A) in a ¹H NMR spectrum. The product may be purified to further extract the bisphenol A to calculate the recovery rate of the diphenol compound. The calculation manner may be referenced to the foregoing paragraphs to perform a comparison of the degradation effects of the foregoing different catalysts. The following embodiments and comparative examples are provided to further describe the degradation reaction of the invention.

Embodiment 2 to Embodiment 15 and Comparative Example 9 to Comparative Example 18 had the same implementation steps with different catalysts used, which were comprehensively described as follows.

Before a degradation reaction was performed, an activation treatment was performed on a synthesized catalyst. The synthesized catalyst was added to a high-pressure reactor, vacuum extraction was performed, and 1 atm of hydrogen was introduced. Then, heating was performed at a temperature of 150°C for 30 minutes to perform an activation. After the activation was complete, the temperature was allowed to return to room temperature to obtain an activated catalyst.

Next, the degradation reaction was performed. 200 mg of the oligomer containing an alkyl aryl ether structure of Formula (A), 2 mL of NMP, and a magnet were added to the foregoing high-pressure reactor containing the catalyst, deoxygenation and dehydration were performed, vacuum extraction was performed, and then 1 atm of hydrogen was introduced. Then, heating was performed to 180°C and stirred for 12 hours. After the reaction was complete, the temperature was allowed to drop to room temperature to obtain a mixture solution. Next, the mixture solution was filtered, and the catalyst was recovered. Afterwards, the filtrate generated after the mixture solution was filtered was distilled and purified to obtain a degradation product. The catalyst types, reaction conditions, and results used in Embodiment 2 to Embodiment 15 and Comparative Example 9 to Comparative Example 18 are shown in Table 2 below.

**Table 2**

| | Catalyst | | Usage Amount (mg) | Degradation Temperature (°C) | Conversion Rate (%) | Diphenol Compound Recovery Rate (%) |
|---|---|---|---|---|---|---|
| Embodiment 2 | Synthesis Example 1 | Fe₁Ni₁Pd₁/CeO₂ | 100 | | 97 | 97 |
| Embodiment 3 | Synthesis Example 2 | Fe₁Ni₁Pt₁/CeO₂ | 100 | | 58 | 57 |
| Embodiment 4 | Synthesis Example 1 | Fe₁Ni₁Pd₁/CeO₂ | 50 | | 81 | 81 |
| Embodiment 5 | Synthesis Example 1 | Fe₁Ni₁Pd₁/CeO₂ | 25 | | 46 | 46 |
| Embodiment 6 | Synthesis Example 5 | Mn₁Ni₁Pd₁/CeO₂ | 100 | | 43 | 42 |
| Embodiment 7 | Synthesis Example 3 | Co₁Ni₁Pd₁/CeO₂ | 100 | | 57 | 56 |
| Embodiment 8 | Synthesis Example 4 | Cu₁Ni₁Pd₁/CeO₂ | 99.8 | | 26 | 26 |
| Embodiment 9 | Synthesis Example 9 | Fe_{0.67}Ni_{0.67}Pd_{0.67}/CeO₂ | 100 | | 86 | 86 |
| Embodiment 10 | Synthesis Example 10 | Fe₁Ni₁Pd_{0.5}/CeO₂ | 100 | | 85 | 85 |
| Embodiment 11 | Synthesis Example 11 | Fe₂Ni₂Pd₁/CeO₂ | 100 | | 94 | 94 |
| Embodiment 12 | Synthesis Example 6 | Fe₁Ni₂Pd₁/CeO₂ | 100 | | 89 | 89 |
| Embodiment 13 | Synthesis Example 7 | Fe₂Ni₁Pd₁/CeO₂ | 100.1 | | 87 | 87 |
| Embodiment 14 | Synthesis Example 8 | Fe₁Ni₁Pd₂/CeO₂ | 100.2 | | 81 | 81 |
| Embodiment 15 | Synthesis Example 12 | Fe₁Ni_{0.5}Pd₁/CeO₂ | 100.1 | 180 | 80 | 80 |
| Comparative Example 9 | Comparative Synthesis Example 1 | Ni₁Pd₁/CeO₂ | 100 | | 94 | 73 |
| Comparative Example 10 | Comparative Synthesis Example 1 | Ni₁Pd₁/CeO₂ | 50.6 | | 58 | 19 |
| Comparative Example 11 | Comparative Synthesis Example 12 | Ni₁Pt₁/CeO₂ | 99.8 | | 14 | 10 |
| Comparative Example 12 | Comparative Synthesis Example 8 | Fe₁Pd₁/CeO₂ | 101.1 | | 44 | 20 |
| Comparative Example 13 | Comparative Synthesis Example 9 | Cu₁Pd₁/CeO₂ | 100.1 | | 24 | 17 |
| Comparative Example 14 | Comparative Synthesis Example 10 | Co₁Pd₁/CeO₂ | 100 | | 42 | 23 |
| Comparative Example 15 | Comparative Synthesis Example 11 | Mn₁Pd₁/CeO₂ | 100.3 | | 40 | 35 |
| Comparative Example 16 | Comparative Synthesis Example 15 | Fe₁Ni₁Pd₅/CeO₂ | 101.2 | | 14 | 9 |
| Comparative Example 17 | Comparative Synthesis Example 13 | Fe₁Ni₅Pd₁/CeO₂ | 100 | | 21 | 13 |
| Comparative Example 18 | Comparative Synthesis Example 14 | Fe₅Ni₁Pd₁/CeO₂ | 100.2 | | 18 | 13 |

It can be seen from Table 2 that for the oligomer containing an alkyl aryl ether structure of Formula (A) serving as the alkyl aryl ether-containing compound, in the same degradation condition, compared to Comparative Example 9 and Comparative Example 12 utilizing a bimetallic catalyst, Example 2 utilizing the trimetallic catalyst of the invention unexpectedly has a higher diphenol compound (BPA) recovery rate in a condition where the conversion rates are similar. This may be attributed to the finding that in a condition where the bimetallic catalyst is utilized, the bisphenol A generated after the alkyl aryl ether structure is cut is easily further degraded to a monophenol by-product, resulting in a high conversion rate but an insufficient diphenol compound recovery rate. In a condition where the trimetallic catalyst of the invention is utilized, the occurrence of the foregoing side reaction may be effectively reduced, improving the diphenol compound recovery rate.

Furthermore, in Embodiment 3 and Comparative Example 11 (replacing Pd with Pt in the catalysts of Embodiment 2 and Comparative Example 9), compared to Comparative Example 11, Embodiment 3 unexpectedly has a higher conversion rate and diphenol compound recovery rate.

Furthermore, in Embodiment 4 and Comparative Example 10 (halving the usage amount of the catalyst of Embodiment 2 and Comparative Example 9), Comparative Example 10 non-desirably results in a significant reduction in conversion rate and diphenol compound recovery rate due to the halved usage amount of the catalyst. Embodiment 4, although resulting in a reduction in conversion rate and diphenol compound recovery rate due to the halved usage amount of the catalyst, may maintain both at 81%. In addition, in Embodiment 5, the catalyst usage amount is further halved. Although the conversion rate is reduced to 46%, the diphenol compound recovery rate may be maintained at 46%, which is still much higher than the result of Comparative Example 10 in terms of diphenol compound recovery rate. This highlights the advantages of the trimetallic catalyst of the invention.

In addition, compared to Comparative Example 13 to Comparative Example 15, since Ni is additionally introduced to each of the catalysts of Embodiment 6 to Embodiment 8 to form trimetallic catalysts, Embodiment 6 may have an unexpected higher diphenol compound recovery rate compared to Comparative Example 15. Embodiment 7 may have an unexpected higher diphenol compound recovery rate compared to Comparative Example 14. Embodiment 8 may have an unexpected higher diphenol compound recovery rate compared to Comparative Example 13. From this, it can be seen that the trimetallic catalyst of the invention indeed surprisingly facilitates the improvement of diphenol compound recovery rate.

In addition, compared to Comparative Example 16 to Comparative Example 18 (where the content of one metal in the trimetallic catalyst is too high, that is, one of x, y, and z exceeds 2), since x, y, and z in the catalysts of Embodiment 10 to Embodiment 16 do not exceed 2, Embodiment 9 to Embodiment 15 have unexpected higher conversion rates and diphenol compound recovery rates. Slight changes in the ratios thereof do not significantly affect the conversion rates and the diphenol compound recovery rates.

### [Comparative Example 19]

Except that the catalyst did not undergo an activation treatment, the remaining steps were the same as Embodiment 3. In Comparative Example 19, since the catalyst did not undergo the activation treatment, the conversion rate was significantly reduced to 19.4%. Due to poor reactivity, diphenol compounds were not able to be obtained (the diphenol compound recovery rate was 0%).

### [Embodiment 16 and Comparative Example 20]

Except for using recovered catalysts from Embodiment 3 and Comparative Example 9, the remaining steps were the same as Embodiment 3. The reaction conditions and results of Embodiment 16 and Comparative Example 20 are shown in Table 3 below.

**Table 3**

| | Catalyst | | Usage Amount (mg) | Degradation Temperature (°C) | Conversion Rate (%) | Diphenol Compound Recovery Rate (%) |
|---|---|---|---|---|---|---|
| Embodiment 16 | Embodiment 3 | Fe₁Ni₁Pd₁/CeO₂ | 99.8 | 180 | 92 | 91 |
| Comparative Example 20 | Comparative Example 9 | Ni₁Pd₁/CeO₂ | 100 | | 93 | 58 |

In Embodiment 16 and Comparative Example 20, the catalysts of Embodiment 2 and Comparative Example 9 were respectively recovered to perform a secondary experiment. It can be seen from Table 3 that in a condition where the recovered catalysts are used, the conversion rates are slightly reduced. However, Embodiment 17 utilizing the trimetallic catalyst of the invention may still have a higher diphenol compound recovery rate. That is, the trimetallic catalyst of the invention unexpectedly has a higher recovery and reuse efficiency.

### [Embodiment 17 and Embodiment 18]

Except for replacing the oligomer containing an alkyl aryl ether structure of Formula (A) with the foregoing epoxy resin cured product (the gel block 1) and the epoxy resin cured product (the gel block 2), the remaining steps were the same as Embodiment 2. The reaction conditions and results of Embodiment 17 and Embodiment 18 are shown in Table 4 below.

**Table 4**

| | Epoxy Resin Cured Product | Catalyst | Usage Amount (mg) | Degradation Temperature (°C) | Conversion Rate (%) | Diphenol Compound Recovery Rate (%) |
|---|---|---|---|---|---|---|
| Embodiment 17 | Gel Block 1 | Fe₁Ni₁Pd₁/CeO₂ | 101.2 | 180 | 94 | 92 |
| Embodiment 18 | Gel Block 2 | Fe₁Ni₁Pd₁/CeO₂ | 100.2 | | 91 | 88 |

Since epoxy resins usually contain alkyl aryl ether structures, the cured products thereof also contain alkyl aryl ether structures. The invention further evaluates the advantageous effect of trimetallic catalysts applied to epoxy resin cured products through Embodiment 17 and Embodiment 18. According to Table 4, under the action of the trimetallic catalyst of the invention, even crosslinked epoxy resin cured products (the gel block 1 and the gel block 2) may still be completely decomposed, and have high conversion rates and high diphenol compound recovery rates.

In a further aspect, the invention relates to the use of the catalyst according to the invention for the recovery of a diphenol compound from alkyl aryl ether-containing compound. As far as the catalyst and the alkyl aryl ether-containing compound are concerned, it applies the same as described above.

## Claims

1. A trimetallic catalyst, comprising a carrier and a first metal M1, a second metal M2, and Ni that are loaded on the carrier, wherein the M1 comprises a transition metal other than Ni, Pd and Pt, the M2 comprises Pd or Pt, and the carrier comprises CeO₂.

2. The trimetallic catalyst according to claim 1, wherein the M1 is Fe, Mn, Co, or Cu.

3. The trimetallic catalyst according to claim 2, wherein the M1 is Fe, and the M2 is Pd or Pt.

4. The trimetallic catalyst according to claim 2, wherein the M1 is Mn, and the M2 is Pd.

5. The trimetallic catalyst according to claim 2, wherein the M1 is Co or Cu, and the M2 is Pd.

6. The trimetallic catalyst according to claim 1, wherein the trimetallic catalyst is represented by Formula (1), and 0<x≦2, 0<y≦2, and 0<z≦2,
(M1)ₓ(Ni)_{y}(M2)_{z}/CeO₂ Formula (1).

7. A method of preparing the trimetallic catalyst according to claim 1, comprising:
dissolving a compound containing the M1, a compound containing the M2, and a Ni-containing compound in water to obtain a mixed solution; and
adding CeO₂ serving as the carrier to the mixed solution to perform reaction.

8. The method of preparing the trimetallic catalyst according to claim 7, wherein the trimetallic catalyst is represented by Formula (1), and x, y, and z respectively represent a millimole number of each of the M1, the M2, and Ni in the compound containing the M1, the compound containing the M2, and the Ni-containing compound used per 23.3 millimoles of a usage amount of the carrier, and 0<x≦2, 0<y≦2, and 0<z≦2,
(M1)ₓ(Ni)_{y}(M2)_{z}/CeO₂ Formula (1).

9. A method for recovering a diphenol compound, comprising:
performing an activation treatment on the trimetallic catalyst according to claim 1 to obtain an activated trimetallic catalyst;
using the activated trimetallic catalyst to perform a degradation treatment on an alkyl aryl ether-containing compound to obtain a mixture solution;
filtering the mixture solution to separate a filtrate and the trimetallic catalyst; and
performing distillation on the filtrate to obtain the diphenol compound.

10. The method for recovering the diphenol compound according to claim 9, wherein the activation treatment comprises:
adding the trimetallic catalyst to a high-pressure reactor, performing vacuum extraction and then introducing hydrogen; and
performing a first heat treatment to increase a temperature to an activation temperature of the trimetallic catalyst, wherein a temperature of the first heat treatment is preferably between 130°C and 300°C.

11. The method for recovering the diphenol compound according to claim 9, wherein the degradation treatment comprises:
adding the alkyl aryl ether-containing compound, the activated trimetallic catalyst, and a solvent to a high-pressure reactor, performing vacuum extraction and then introducing hydrogen; and
performing a second heat treatment to increase a temperature to a degradation temperature of the alkyl aryl ether-containing compound, wherein preferably the second heat treatment is between 150°C and 240°C.

12. The method for recovering the diphenol compound according to claim 9, wherein the alkyl aryl ether-containing compound comprises a monomer containing an alkyl aryl ether structure, an oligomer containing an alkyl aryl ether structure, a polymer containing an alkyl aryl ether structure, or a combination thereof.

13. The method for recovering the diphenol compound according to claim12, wherein the polymer containing alkyl aryl ether groups comprises a thermosetting polymer or a thermoplastic polymer.

14. The method for recovering the diphenol compound according to claim9, wherein in the degradation treatment, taking a total amount of the alkyl aryl ether-containing compound as a reference, the activated trimetallic catalyst is added in an amount between 20 wt% and 70 wt%.

15. Use of a catalyst according to any one of claims 1 to 6 for the recovery of a diphenol compound from an alkyl aryl ether-containing compound.
